# EUROPÄISCHE PATENTANMELDUNG

(11) **EP 1 086 955 A1**
(43) Veröffentlichungstag der Anmeldung: **28.03.2001**
(21) Anmeldenummer: 99118631.3
(22) Anmeldetag: 21.09.1999
(51) Int. Cl.: C07K 14/705, C07K 7/08, A61P 37/00

(54) **Peptide gegen DCM hervorrufende Autoantikörper**

(71) Anmelder: Affina Immuntechnik GmbH, 12489 Berlin (DE)
(72) Erfinder: Rönspeck, Wolfgang, 10715 Berlin (DE); Kunze, Rudolf, Dr., 17268 Stegelitz (DE); Wallukat, Gerd, Dr., 13129 Berlin (DE); Dierenfeld, Manuela, 15827 Blankenfelde (DE)
(74) Vertreter: Meyers, Hans-Wilhelm, Dr.Dipl.-Chem.

(57) **Zusammenfassung**

Es werden Peptide beschrieben, die Autoantikörper, die mit Dilatativer Cardiomyopathie (DCM) in kausal pathologischer Verbindung stehen. Die Peptide können z.B. an eine feste Phase gebunden werden. Durch Behandlung von Blut der Patienten mit DCM und den erfindungsgemäßen Peptiden, können Autoantikörper entfernt werden.

## Beschreibung

Gegenstand der vorliegenden Erfindung sind Peptide gegen DCM hervorrufende Autoantikörper, Arzneimittel enthaltend diese Peptide, Verwendung der Peptide, Verfahren zur Behandlung von mit β₁-adrenerg aktiven Autoantikörpern in Verbindung stehenden Erkrankungen sowie eine Vorrichtung zur Immunadsorption enthaltend, die an eine Festphase gebundenen Peptide.

Das Immunsystem ist ein essentieller Bestandteil bei allen tierischen Lebewesen. Bei den Säugetieren dient es insbesondere zur Abwehr von Mikroorganismen, zur Gewebesregeneration und zur Vernichtung von Tumorzellen. In der klassischen Immunologie wird unterschieden zwischen einer zellulären und einer humoralen Immunabwehr. Darunter versteht man zwei unterscheidbare, aber miteinander kooperierende Systeme, die letztlich das Immunsystem darstellen.

Es existieren eine Reihe von Krankheiten, die auf Grund ihrer Pathogenese als Autoimmunerkrankungen angesehen werden. Bei solchen Krankheiten richtet sich das Immunsystem der Betroffenen gegen eigene Organe, Gewebe, Zellen oder Proteine u.a. Moleküle. Zu den vorwiegend zellvermittelten Autoimmunerkrankungen gehören Multiple Sklerose und Diabetes (Typ I).

Eine zweite Gruppe bilden die vorzugsweise antikörpervermittelten Autoimmunerkrankungen. Zu ihnen zählen beispielsweise Rheuma, die seltener vorkommenden Autoimmunerkrankungen wie Myasthenia gravis oder Lupus erythematodes und neuerdings auch die Dilatative Cardiomyopathie (DCM).

Die Pathogenese der meisten Autoimmunerkrankungen ist unbekannt. Es gibt verschiedene Hypothesen und Modelle, wie man die Entstehung von Autoimmunerkrankungen erklären kann. Ein Erklärungsmodell stellt das antigene/molekulare Mimikry dar. Hierbei geht man davon aus, dass Mikroorganismen, z.B. Viren oder Parasiten, sich mit bestimmten Molekülen ausstatten, die z.B. wirtseigenen Strukturen täuschend ähnlich oder sogar teilweise mit ihnen identisch sind und folglich vom Immunsystem des Wirtes nicht erkannt werden.

Werden sie allerdings als fremd erkannt und werden gegen sie Antikörper produziert, erkennen diese Antikörper dann körpereigene, ähnliche Strukturen, mit der Folge, dass das Immunsystem und das Komplementsystem aktiviert werden. Dieses löst dann vor Ort pathologische Reaktionen im Gewebe - z.B. chronische Entzündungen - aus oder aber es kommt zu einer pathologischen Fehlfunktion der Zellen, an denen die Autoantikörper gebunden haben.

Als ein herausragendes Beispiel hierfür kann die Dilatative Cardiomyopathie gelten. Bei dieser Autoimmunerkrankung bildet der Organismus fehlerhaft Autoantikörper die an definierte Bereiche des β₁-adrenergen Rezeptors binden. Diese Bereiche befinden sich auf dem ersten und zweiten Loop von insgesamt drei extrazellulären Loops des β₁-adrenergen Rezeptors.

Solche Autoantikörper, die in der Lage sind, an diese Bereiche zu binden, erzeugen in biologischen Tests an Ratten-Cardiomyozyten in der Zellkultur (diese Zellen haben einen nahezu identischen β₁-adrenergen Rezeptor auf der Oberfläche) eine Erhöhung der Pulsationsrate. Man spricht hier von einer pharmakoaktiven, dem Adrenalin ähnlichen Wirkung der Autoantikörper. Die Autoantikörper, die gegen die Epitope auf den Loops 1 und 2 des β₁-adrenergen Rezeptors gerichtet sind, werden vor allem bei Patienten mit DCM beobachtet. Gelegentlich werden solche Autoantikörper auch bei Patienten mit Herzrythmusstörungen und Myocarditis beobachtet.

Die Dilatative Cardiomyopathie ist eine Autoimmunerkrankung, die unbehandelt zu einer starken Beeinträchtigung der Herzleistung - Reduktion der Pumpleistung bei gleichzeitiger Ausdehnung des Herzmuskelgewebes durch Infiltrate - und zur Herztransplantation oder zum Tod führt.

Werden allerdings dem Patienten durch eine Blutwäsche die Antikörper aus dem Blut entfernt, kommt es im Laufe eines Jahres zu einer Regeneration des Herzmuskels und zu einer drastischen Verbesserung der Herzmuskelleistung, die beinahe wieder die Werte von gesunden Personen erreicht.

Bei Patienten mit DCM lässt sich aus dem Plasma eine Immunglobulinfraktion isolieren, welche die spezifische Autoantikörper enthält, die an den β₁-adrenergen Rezeptor binden und darüber die Zelle aktivieren. Fügt man der Zellkultur von Rattencardiomyocyten Peptide des β₁-adrenergen Rezeptors hinzu, welche die Bindungsstelle für die Autoantikörper darstellen, lässt sich der pathologische Effekt der Immunglobulinfraktion aufheben.

Werden dieselben Peptide, welche den nativen Sequenzen entsprechen an eine Festphase gekoppelt, sind sie nicht in der Lage, aus dem Blutplasma eines Patienten die beschriebenen Autoantikörper zu binden und zu eliminieren. D.h. das Peptid, die der nativen Sequenz der β₁-adrenergen Rezeptors entsprechen und Bindungsstellen für die beschriebenen pathologischen Autoantikörper darstellen nicht für die Immunadsorption zu verwende sind.

Ein der Erfindung zu Grunde liegendes technisches Problem besteht darin, Peptide bereitzustellen, die pathologische Autoantikörper, die gegen funktionelle Epitope gerichtet sind aus dem Blut oder Plasma von Patienten mit positivem Antikörperstatus oder DCM erkennen, binden und eliminieren und wobei die Peptide gleichzeitig neben dem jeweils die Antikörperwirkung neutralisierenden Epitope noch Aminosäuresequenzen enthalten, die eine Bindung der pathologischen Antikörper ermöglichen.

Gelöst wird das Problem überraschenderweise durch Peptide mit der Aminosäuresequenz
X01-X02-X03-G-X04-X05-X06-X07-X08-X09-W-X10-X11-X12
wobei
X01 = Aminogruppe, Acetylgruppe, Biotingruppe, Fluoreszenzmarker, Spacer, Linker oder Deletion
X02 = D,G,E,T, S oder Deletion,
X03 = W,Y,F,G,T
X04 = T,S,A,G
X05 = V,I,W,F,Y
X06 = L,F,Y,W
X07 = S,A,C
X08 = G,D,E,N,Q
X09 = F,L,I,Y,
X10 = E,Q,T,S,L
X11 = Y,F,T,S,W
X12 = Amid, GKK, oder ein Spacer ist
und Peptide mit der Aminosäuresequenz
X01-X02-W-X03-R-X04-X05-X06-X07-X08-E-A-R-X09-X10-X11-X12-X13-X14-X15-X16-X17
wobei
X01 = Aminogruppe, Aminosäure, Peptid, Acetylgruppe, Biotingruppe, Fluoreszenzmarker, Spacer, Linker oder Deletion
X02 = H, E, Q,
X03 = H, F, Y, W
X04 = A, V
X05 = G, T, E, S, D, N
X06 = S, H, A
X07 = D, N ,Q, E
X08 = G, A, oder eine Deletion
X09 = D, N, R
X10 = S, T, C, M
X11 = H F,W,Y
X12 = A, D, N, S
X13 = D, N
X14 = E, P
X15 = R, K, T
X16 = S, T, C, M oder eine Deletion
X17 = Amid, GKK, SGKK oder ein Spacer ist.

Bevorzugt werden die folgenden Peptide eingesetzt:
TGSFF SELWT SGKK-amid, (Seq. ID. No 1);
Ac-E YGSFF SELWT SGKK-amid, (Seq. ID. No 2);
Ac-T YGTLF SDFWL SGKK-amid, (Seq. ID No. 3);
Ac- His-Trp-Trp-Arg-Ala-Glu-Ser-Asp-----Glu-Ala-Arg-Arg-Ser-Tyr-Asn-Asp-Pro-Lys-Amid, (Seq. ID No. 4);
Ala-Arg-Arg-Cys-Tyr-Asn-Asp-Pro-Lys-Amid (Seq. ID. No. 5)

Die erfindungsgemäßen Peptide werden insbesondere von Antikörpern von Patienten mit Dilatativer Cardiomyopathie gebunden.

Erfindungsgemäß können als Linker alle Strukturen, die zu diesem Zweck verfügbar sind, eingesetzt werden, solange diese nicht negativ mit dem Bindungsverhalten der Peptide gegenüber den Antikörpern interferieren. Ein Linker ist üblicherweise eine chemische Verbindung, welche mindestens eine Verknüpfungsstelle (funktionelle Gruppe) an einer ansonsten funktionslosen polymeren Matrix bereitstellt.

Die Verknüpfungsstelle dient zur Kopplung eines Liganden oder eines Spacers und korrespondiert mit den chemischen Eigenschaften des Liganden oder Spacers. Diese Verknüpfung ist in Abhängigkeit von der Art des Linkermoleküls stabil oder spaltbar.

Ein Ligand ist üblicherweise eine Verbindung mit einer besonderen Eigenschaft. Erfindungsgemäß bevorzugt handelt es sich um ein Peptid, welches in der Lage ist, spezifisch einen Autoantikörper zu binden, der eine adrenerge Wirkung besitzt und gegen den β1-adrenerger-Rezeptor des Herzmuskels gerichtet ist.

Erfindungsgemäß werden die folgenden Linker bevorzugt eingesetzt:

α-Aminocarbonsäuren sowie deren Homo- und Heterooligomere, α,ω-Aminocarbonsäuren sowie deren verzweigte Homo- oder Heterooligomere, sonstige Aminosäuren sowie die linearen und verzweigten Homo- oder Heterooligomere (Peptide); Amino-oligoalkoxy-alkylamine; Maleinimidocarbonsäure- Derivate; Oligomere von Alkylaminen; 4-Alkylphenyl-Derivate; 4-Oligoalkoxyphenyl- oder 4-Oligoalkoxyphenoxy-Derivate; 4-Oligoalkylmercaptophenyl- oder 4-Oligoalkylmercaptophenoxy-Derivate; 4-Oligoalkylaminphenyl- oder 4-Oligoalkylaminyphenoxy-Derivate; (Oligoalkylbenzyl)-phenyl- oder 4-Oligoalkylbenzyl)-phenoxy-Derivate sowie 4-Oligoalkoxybenzyl)-phenyl- oder 4-Oligoalkoxybenzyl)-phenoxy-Derivate; Trityl-Derivate; Benzylxyaryl- oder Benzyloxyalkyl-Derivate; Xanthen-3-yl-oxyalkyl-Derivate; (4-Alkylphenyl)- oder ω-(4-Alkylphenoxy)-alkansäure-Derivate; Oligoalkyl-phenoxyalkyl- oder Oligoalkoxy-phenoxyalkyl-Derivate; Carbamat-Derivate; Amine; Trialkylsilyl- oder Dialkyl-alkoxysilyl-Derivate; Alkyl- oder Aryl-Derivate und Kombinationen davon.

Zum Einsatz der erfindungsgemäßen werden diese insbesondere an eine Festphase gebunden Vorzugsweise erfolgt die Bindung der Peptide über einen Spacer an die Festphase. Als Spacer kommen praktisch alle für diese Funktion geeigneten chemischen Verbindungen oder Gruppen in Betracht, so lange sie nicht das Bindungsverhalten so negativ beeinflussen, dass eine Bindung des Antikörpers mit dem Peptid verhindert oder wesentlich beeinträchtigt wird.

Ein Spacer ist üblicherweise eine Verbindung, die falls nötig zwischen Ligand und Linker eingebaut wird und dazu dient, den Liganden im für die Bindung des Autoantikörpers richtigen Abstand und richtigen räumlichen Lage zu positionieren. Spacer sind Moleküle mit mindestens zwei chemisch aktiven Gruppen (fünktionelle Gruppen), wovon eine Gruppe an das Linkermolekül bindet und mindestens eine zweite funktionelle Gruppe die Bindung zu einem Liganden herstellt. Durch die Auswahl des Spacers kann je nach Bedarf eine Erhöhung der Flexibilität sowie eine Verbesserung der Zugänglichkeit als auch eine gerichtete Anordnung der Liganden und Erhöhung der Ligandendichte auf der Oberfläche erreicht werden.

Spacer sind z.B. ω-Aminocarbonsäuren sowie deren Homo- und Heterooligomere, α,ω -Aminocarbonsäuren sowie deren verzweigte Homo- oder Heterooligomere, sonstige Aminocarbonsäuren sowie deren lineare und verzweigte Homo- oder Heterooligomere, Maleinimidocarbonsäurederivate, Hydroxycarbonsäurederivate, Dicarbonsäurederivate, Diaminderivate, Dihydroxyalkylderivate und Hydroxyalkylaminderivate. Vorzugsweise werden Mono- oder Dioligomere von β-Alanin oder ω-Aminohexansäure und verzweigte Mono- oder Dioligomere von Lysin oder Ornithin verwendet.

In einer anderen Ausführungsform der Erfindung, werden die erfindungsgemäßen Peptide als Arzneimittel eingesetzt.

Bei dieser Konzeption werden Peptide in einer besonderen Weise so verändert (z.B. durch Zyklisierung), dass sie durch Serumproteasen nicht zerstört werden können und in Lösungen Antikörper binden. Auf diese Weise kann eine in vivo-Neutralisation der Antikörper stattfinden, in dem man die entsprechend aufbereiteten Peptide intravenös verabreicht. Die Peptide sind hier als Arzneimittel aufzufassen. Ihre Entwicklung leitet sich direkt aus den die antikörperbindenden Peptiden, welche säulenmatrixfixiert sind, ab.

Die Menge der zu verabreichenden Peptide hängt dabei von ihrem Molekulargewicht (also ihrer Größe) sowie von der Konzentration von in der Blutbahn und anderen Kompartments erreichbaren Autoantikörpern ab. Nach bisherigem Kenntnisstand bewegen sich die Mengen von Autoantikörpern in µg und ng-Bereich. Eine Menge zwischen 1-5 µg Peptid sollte ausreichen die vorhandenen Antikörper zu binden und diese einer Elimination als Immunkomplex entsprechend der natürlichen Clearencemechanismen zu zuführen. In der Folge kann das niedriger dosiert werden, müssen doch nur nachgebildete Antikörper eliminiert werden.

Prinzipiell sind auch hier andere Darreichungsformen möglich. Bei Anwendung entsprechender galenischer Verfahren sollte eine Resorption von β1-antikömerbindenden Peptiden erreicht werden Hier wären die Dosierungen dann allerdings erheblich, etwa um den Faktor 10 bis 20, höher.

Die erfindungsgemäßen Peptide können zur Herstellung eines Arzneimittels zur Behandlung mit von mit β₁-adrenerg aktiven Autoantikörpern in Verbindung stehenden Erkrankungen, insbesondere Dilatative Cardiomyopathie, Bluthochdruckerkrankungen und eine durch Trypanosoma cruzi induzierte Form der Cardiomyopathie eingesetzt werden. Neben der Dilatativen Cardiomyopathie existieren noch eine Reihe von Erkrankungen, die den Autoimmunerkrankungen zugeordnet werden können und die ähnlichen Pathomechanismen unterliegen. Für die Präeklampsie und bestimmte Formen des malignen Bluthochdrucks wurden ebenfalls Autoantikörper beschrieben, welche durch eine Stimulation des Angiotensin-Rezeptors bzw. des α₁-Rezeptors von Zellen zu deren Überaktivierung beitragen und die an der Entstehung von definierten Krankheitsbildern beteiligt sind. Die Anwendung von Peptiden zur Elimination von diesen speziellen Autoantikörpern bzw. zur in vivo-Neutralisation der Autoantikörper ist hier in Analogie zur Dilatativen Cardiomyopathie zu sehen.

Erfindungsgemäß beansprucht wird ein Verfahren zur Behandlung von mit β₁-adrenerg aktiven Autoantikörpern in Verbindung stehenden Erkrankungen durch Entfernung der Autoantikörper mittels von an eine Festphase gebundenen Peptiden. Dies kann vorteilhafterweise mit einer erfindungsgemäßen Vorrichtung zur Chromatographie enthaltend die an eine Festphase gebundenen erfindungsgemäßen Peptide erfolgen.

Die Peptide befinden sich festphasenfixiert, z.B. an Sepharose, in einem geschlossenen, sterilen Behälter, der in der Regel zwischen 5 und 250ml Volumen hat. In diesem Sterilraum fließt das Blutplasma der Patienten, von dem zuvor die Zellen durch eine medizintechnische Apparatur entfernt worden sind, über bzw. durch die Adsorptionsmatrix, d. h. die peptidbeschichteten Sepharose-Oberfläche. Hierbei kommt es zu einer Bindung der pathologischen Autoantikörper an die Peptide, welche Regionen des β₁-adrenergen Rezeptors simulieren. Sofern geeignete Adsorptionsmatrices eingesetzt werden, kann auf eine vorherige Abtrennung der Zellen verzichtet werden.

Die restlichen Bestandteile des Blutplasmas oder Blutes, so auch alle notwendigen und nützlichen Immunglobuline verlassen dann die Säule und werden dem Patienten wieder in die Blutbahn gegeben. Diese Vorrichtung zur extrakorporalen Therapie ist Stand der Technik so weit es dabei um eine unspezifische Elimination von Plasmaproteinen oder Immunglobulin geht.

Die erfindungsgemäße Einrichtung bedient sich der Peptide, die vom β₁-adrenergen Rezeptor abgeleitet worden sind, um die kleine aber pathologisch relevante Autoantikörperfraktion aus dem Plasma zu entfernen.

Nach dem Durchfluss einer bestimmten Menge an Blutplasma kann der Blutplasmastrom auf eine zweite technisch identische Säule umgeleitet werden, während die erste Säule regeneriert wird. D. h. die beladenen Antikörper werden vom Peptid abgetrennt und verworfen, indem verschiedene Spül- und Elutionslösungen, vorzugsweise physiologischer Kochsalzlösung mit und ohne zusätzliche Pufferung beispielsweise durch Phosphat, Glycin oder Citrat und einen pH-Bereich von pH 2 bis pH 7,5 verwendet werden. Die auf diese Weise wieder regenerierte Säule steht anschließend erneut für die Bindung und Elimination der pathologischen Autoantikörper aus dem Blutplasma des Patienten zur Verfügung. Dieses Doppelsäulenprinzip hat sich bewährt und wird immer dort angewendet wo eine Regeneration der Säule erforderlich ist.

Eine zweite Variante der Anwendung von Peptiden zur Elimination pathologischer Autoantikörper beinhaltet die Verwendung von Einmalsäulen. Bei diesen Säulen befindet sich soviel Peptid auf der Festphasenmatrix, daß in einer mehrstündigen Behandlung große Teile der Autoantikörper aus dem Plasma entfernt werden können. Der Vorteil liegt hier in dem Verzicht auf die zeitaufwendige und umständliche Regeneration der Adsorptionsmatrix.

Eine dritte Variante der Behandlung von DCM-Patienten durch Elimination pathologischer Antikörper aus dem Blutplasma liegt in der Verwendung von Säulen, bei denen aufgrund ihrer Beschaffenheit eine vorherige Trennung von Plasma und Blutzellen nicht erforderlich ist.

Für die Verwendung der Säulen sind technische Vorrichtungen erforderlich, welche durch verschiedene Schläuche, Pumpen, Monitore und andere Überwachungsysteme einen der Behandlung adäquaten Blut- bzw. Plasmazufluss und -durchfluss garantieren.

SEQUENZPROTOKOLL

<110> Affina Immuntechnik

<120> Peptide gegen DCM hervorrufende Autoantikörper

<130> 991891de Affina DCM

<140>
<141>

<160> 5

<170> PatentIn Ver. 2.1

<210> 1
<211> 14
<212> PRT
<213> Homo sapiens

<400> 1
Thr Gly Ser Phe Phe Ser Glu Leu Trp Thr Ser Gly Lys Lys
  1               5                  10

<210> 2
<211> 15
<212> PRT
<213> Homo sapiens

<400> 2
Glu Tyr Gly Ser Phe Phe Ser Glu Leu Trp Thr Ser Gly Lys Lys
  1               5                  10                  15

<210> 3
<211> 15
<212> PRT
<213> Homo sapiens

<400> 3
Thr Tyr Gly Thr Leu Phe Ser Asp Phe Trp Leu Ser Gly Lys Lys
  1               5                  10                  15

<210> 4
<211> 18
<212> PRT
<213> Homo sapiens

<400> 4
His Trp Trp Arg Ala Glu Ser Asp Glu Ala Arg Arg Ser Tyr Asn Asp
1               5                   10                  15

Pro Lys

<210> 5
<211> 9
<212> PRT
<213> Homo sapiens

<400> 5
Ala Arg Arg Cys Tyr Asn Asp Pro Lys
1               5

### Annex zu den Anmeldungsunterlagen - nachgereichtes Sequenzprotokoll

SEQUENZPROTOKOLL

<110> Affina Immuntechnik

<120> Peptide gegen DCM hervorrufende Autoantikörper

<130> 992091ep Affina DCM

<140> 99118631.3
<141> 1999-09-21

<160> 7

<170> PatentIn Ver. 2.1

<210> 1
<211> 14
<212> PRT
<213> Homo sapiens

<400> 1
Thr Gly Ser Phe Phe Ser Glu Leu Trp Thr Ser Gly Lys Lys
  1               5                  10

<210> 2
<211> 15
<212> PRT
<213> Homo sapiens

<400> 2
Glu Tyr Gly Ser Phe Phe Ser Glu Leu Trp Thr Ser Gly Lys Lys
  1               5                  10                  15

<210> 3
<211> 15
<212> PRT
<213> Homo sapiens

<400> 3
Thr Tyr Gly Thr Leu Phe Ser Asp Phe Trp Leu Ser Gly Lys Lys
  1               5                  10                  15

<210> 4
<211> 18
<212> PRT
<213> Homo sapiens

<400> 4
His Trp Trp Arg Ala Glu Ser Asp Glu Ala Arg Arg Ser Tyr Asn Asp
1               5                   10                  15

Pro Lys

<210> 5
<211> 9
<212> PRT
<213> Homo sapiens

<400> 5
Ala Arg Arg Cys Tyr Asn Asp Pro Lys
1               5

<210> 6
<211> 14
<212> PRT
<213> Homo sapiens

<400> 6
Xaa Xaa Xaa Gly Xaa Xaa Xaa Xaa Xaa Xaa Trp Xaa Xaa Xaa
1               5                   10

<210> 7
<211> 22
<212> PRT
<213> Homo sapiens

<400> 7
Xaa Xaa Trp Xaa Arg Xaa Xaa Xaa Xaa Xaa Glu Ala Arg Xaa Xaa Xaa
1               5                   10                  15

Xaa Xaa Xaa Xaa Xaa Xaa
            20

## Patentansprüche

1. Peptide mit der Aminosäuresequenz
X01-X02-X03-G-X04-X05-X06-X07-X08-X09-W-X10-X11-X12
wobei
X01 = Aminogruppe, Acetylgruppe, Biotingruppe, Fluoreszenzmarker, Spacer, Linker oder Deletion
X02 = D,G,E,T, S oder Deletion,
X03 = W,Y,F,G,T
X04 = T,S,A,G
X05 = V,I,W,F,Y
X06 = L,F,Y,W
X07 = S,A,C
X08 = G,D,E,N,Q
X09 = F,L,I,Y,
X10 = E,Q,T,S,L
X11 = Y,F,T,S,W
X12 = Amid, GKK, oder ein Spacer ist
und Peptide mit der Aminosäuresequenz
X01-X02-W-X03-R-X04-X05-X06-X07-X08-E-A-R-X09-X10-X11-X12-X13-X14-X15-X16-X17
wobei
X01 = Aminogruppe, Aminosäure, Peptid, Acetylgruppe, Biotingruppe, Fluoreszenzmarker, Spacer, Linker oder Deletion
X02 = H, E, Q,
X03 = H, F, Y, W
X04 = A, V
X05 = G, T, E, S, D, N
X06 = S, H, A
X07 = D, N ,Q, E
X08 = G, A, oder eine Deletion
X09 = D, N, R
X10 = S, T, C, M
X11 = H F,W,Y
X12 = A, D, N, S
X13 = D, N
X14 = E, P
X15 = R, K, T
X16 = S, T, C, M oder eine Deletion
X17 = Amid, GKK, SGKK oder ein Spacer ist.

2. Peptide nach Anspruch 1, dadurch gekennzeichnet, dass es sich um
TGSFF SELWT SGKK-amid,
Ac-E YGSFF SELWT SGKK-amid,
Ac-T YGTLF SDFWL SGKK-amid,
Ac- His-Trp-Trp-Arg-Ala-Glu-Ser-Asp-----Glu-Ala-Arg-Arg-Ser-Tyr-Asn-Asp-Pro-Lys-Amid.
Ala-Arg-Arg-Cys-Tyr-Asn-Asp-Pro-Lys-Amid
handelt.

3. Peptide nach Anspruch 1 oder 2, dadurch gekennzeichnet, dass die Peptide von Antikörpern von Patienten mit Dilatativer Cardiomyopathie gebunden werden.

4. Peptide nach einem der Ansprüche 1 bis 3, dadurch gekennzeichnet, dass der Linker ausgewählt wird aus der Gruppe, bestehend aus
- α-Aminocarbonsäuren sowie deren Homo- und Heterooligomere
- α,ω-Aminocarbonsäuren sowie deren verzweigte Homo- oder Heterooligomere
- sonstige Aminosäuren sowie die linearen und verzweigten Homo- oder Heterooligomere (Peptide)
- Amino-oligoalkoxy-alkylamine
- Maleinimidocarbonsäure- Derivate
- Oligomere von Alkylaminen
- 4-Alkylphenyl-Derivate
- 4-Oligoalkoxyphenyl- oder 4-Oligoalkoxyphenoxy-Derivate
- 4-Oligoalkylmercaptophenyl- oder 4-Oligoalkylmercaptophenoxy-Derivate
- 4-Oligoalkylaminphenyl- oder 4-Oligoalkylaminyphenoxy-Derivate
- (Oligoalkylbenzyl)-phenyl- oder 4-(Oligoalkylbenzyl)-phenoxy-Derivate sowie 4-(Oligoalkoxybenzyl)-phenyl- oder 4-(Oligoalkoxybenzyl)-phenoxy-Derivate
- Trityl-Derivate
- Benzyloxyaryl- oder Benzyloxyalkyl-Derivate
- Xanthen-3-yl-oxyalkyl-Derivate
- (4-Alkylphenyl)- oder ω-(4-Alkylphenoxy)-alkansäure-Derivate
- Oligoalkyl-phenoxyalkyl- oder Oligoalkoxy-phenoxyalkyl-Derivate
- Carbamat-Derivate
- Amine
- Trialkylsilyl- oder Dialkyl-alkoxysilyl-Derivate
- Alkyl- oder Aryl-Derivate
- und Kombinationen davon.

5. Peptide nach einem der Ansprüche 1 bis 4, dadurch gekennzeichnet, dass die Peptide an eine Festphase gebunden sind.

6. Peptide nach einem der Ansprüche 1 bis 5, dadurch gekennzeichnet, dass die Peptide über den Spacer an eine Festphase gebunden sind.

7. Arzneimittel enthaltend die Peptide nach einem der Ansprüche 1 bis 6.

8. Verwendung der Peptide nach einem der Ansprüche 1 bis 6 zur Herstellung eines Arzneimittels zur Behandlung mit von mit β₁-adrenerg aktiven Autoantikörpern in Verbindung stehenden Erkrankungen, insbesondere Dilatative Cardiomyopathie.

9. Verfahren zur Behandlung von mit β₁-adrenerg aktiven Autoantikörpern in Verbindung stehenden Erkrankungen durch Entfernung der Autoantikörper mittels von an eine Festphase gebundenen Peptiden nach Anspruch 5 oder 6.

10. Vorrichtung zur Chromatographie enthaltend an eine Festphase gebundene Peptide nach Anspruch 5 oder 6.
